# EUROPÄISCHE PATENTSCHRIFT

(11) **EP 3 940 451 B1**
(45) Veröffentlichungstag und Bekanntmachung des Hinweises auf die Patenterteilung: **26.02.2025**
(21) Anmeldenummer: 21185742.0
(22) Anmeldetag: 15.07.2021
(51) Int. Cl.: G02C 7/16, A61F 9/02, A61F 9/04, G02C 11/00, G02C 5/22, A63B 69/00, G02C 5/00, G02C 9/02

(54) **SHIELDING DEVICE FOR COVERING THE LOWER FIELD OF VISION**
VORRICHTUNG ALS SICHTBLENDE ZUM ABDECKEN DES UNTEREN SICHTFELDES
DISPOSITIF SERVANT D'ÉCRAN POUR COUVRIR LE CHAMP DE VISION INFÉRIEUR

(30) Priorität: 15.07.2020 CH 8742020
(43) Veröffentlichungstag der Anmeldung: 19.01.2022
(73) Patentinhaber: Nikolic, Mihailo, 6390 Engelberg (CH)
(72) Erfinder: NIKOLIC, Mihailo, 6390 Engelberg (CH)
(74) Vertreter: Zwick, Evelyn

(56) Entgegenhaltungen:
- ES-A2- 2 663 126
- FR-A1- 2 978 913
- US-A- 1 152 431
- US-A- 2 721 322
- US-A- 3 613 116
- US-A- 3 660 852
- US-A- 4 022 466
- US-A- 4 916 754
- US-A- 593 374

## Beschreibung

### Technisches Gebiet

Die Erfindung betrifft eine am Kopf tragbare Vorrichtung als Sichtblende zum Abdecken des unteren Sichtfeldes, ungeachtet der aktuellen Kopfneigung, welche einem Benutzer beim Tragen beispielsweise die Sicht auf eine Schreibtastatur oder zu den Tasten eines Klaviers verdeckt. Die Vorrichtung umfasst ein wie ein Brillengestell tragbares Traggestell sowie eine flächig ausgestaltete Blende.

### Stand der Technik

Es ist bekannt, dass Klavierspieler oder Tastaturschreiber, welche die jeweilige Tätigkeit einüben möchten, dafür nicht nach unten schauen sollten. Sie sollten dafür das Notenblatt resp. das Vorlageblatt oder den Bildschirm sehen, nicht aber die Hände bei der Tastatur oder bei den Tasten des Klaviers.

Selbst bei gutem Willen der Schüler kann nicht vermieden werden, dass der Blick hin und wieder kurzzeitig nach unten wandert. Dadurch ist die Stelle auf dem Notenblatt oder dem Vorlageblatt verloren gegangen und muss wiedergefunden werden, was eine Unterbrechung im Arbeitsfluss darstellt.

Aus der ES2663126 sind Brillen für Ballsportler bekannt, beispielsweise für Basketballer, die ebenfalls nicht nach unten schauen sollten, wenn sie drippeln. Solche Brillen weisen eine Blende im unteren Sichtfeld auf. Es hat sich gezeigt, dass solche Vorrichtungen für den Zweck des Tastaturübens, hier sowohl Klaviertasten als auch Texte eintippen umfassend, nicht geeignet sind. Zu einfach kann der Kopf genügend geneigt werden, wodurch die jeweiligen Tasten sichtbar werden. Ähnliche Ausführungen für Eishockey Spieler sind in der US4022466 beschrieben.

In der US 1152431 ist ein Brillengestell beschrieben, an dem eine Blende unterhalb des Augenbereichs angeordnet ist, welche die Sicht nach unten abdeckt, solange der Blick gerade nach vorne gerichtet ist.

Zudem wird in der US 593374 A eine Schirmmütze offenbart mit einer zusätzlichen Blende, welche in einer ersten Position unten am Schirm der Mütze anliegt und in einer zweiten Position nach unten geklappt werden kann, sodass sie auf der Nase anliegt, um zu verhindern, dass am Boden reflektierte Strahlung zu den Augen gelangen kann.

Eine andere Vorrichtung als Blende zum Klavierspielen für Schüler ist aus belastbarem Papier ausgestaltet und stützt sich einseitig auf den Schultern des Schülers ab und auf der anderen Seite auf dem Klavier, beispielsweise im Bereich der Notenauflage. Diese Vorrichtung ist sehr sperrig und unangenehm zu tragen. Zudem sind die Tasten der ganz tiefen und der ganz hohen Töne in der Regel stets noch sichtbar.

Ein weiterer Nachteil besteht darin, dass der Schüler die Noten nicht mehr umblättern kann, da er die Arme nur umständlich seitlich an der Blende vorbei zu den Notenblättern führen kann. Zum anderen werden die Schultern und Arme, die beim Klavierspielen frei beweglich bleiben müssen, blockiert, und auch die Hände werden in ihrer vertikalen Bewegungsfreiheit eingeschränkt, was beim Klavierspielen ein essentielles Hindernis darstellt.

### Darstellung der Erfindung

Es ist die Aufgabe der vorliegenden Erfindung, eine Vorrichtung zu beschreiben, mit welcher ein Tastaturschüler bei keinem Neigungswinkel des Kopfes auf die Tasten sehen kann, und die dennoch komfortabel tragbar ist.

Die Aufgabe wird gelöst durch die Merkmale des unabhängigen Patentanspruchs. Weitere vorteilhafte Ausführungen sind in den Unteransprüchen angegeben.

Erfindungsgemäss ist bei einer eingangs erwähnten Vorrichtung die Blende beidseitig seitlich am Traggestell schwenkbar an Gelenken angebracht und steht unterhalb der Augenbereiche nach vorne, also in Sehrichtung, ab. Die Gelenke sind derart ausgestaltet, dass sie bei einer Neigung des Traggestells eine Neigung der Blende in Gegenrichtung erzwingen.

Vorzugsweise sind hinter den Gelenken, demnach entgegen der Sehrichtung, an der Blende Gegengewichte angebracht. Indem ihr Abstand zu den Gelenken verstellt wird, kann die Grundneigung der Blende eingestellt werden. Zudem können die Gelenke selbst an neigbaren Haltevorrichtungen angebracht sein, um die gewünschte Grundeinstellung des Neigungswinkels der Blende einzustellen. Zudem kann das Sichtfeld weiter eingestellt werden, wenn die vordere Kante der Blende in variablem Abstand zum Traggestell positionierbar ist.

Die Gelenke können auf verschiedene Arten ausgestaltet sein. Einerseits können sie durch zwei sich kreuzende Stege ausgestaltet sein, an deren oberen Enden das Traggestell und an deren unteren Enden die Blende befestigt ist. Andererseits kann ein Distanzhalter den Tragbügel mit der Blende beidseits schwenkbar verbinden. Als Führung, welche die erforderliche Gegenneigung der Blende bei Neigung des Traggestells erzwingt, können bei den Anbringungen der Distanzhalter entweder Zahnradsegmente oder Hebel jeweils am Traggestell und an der Blende drehfest angebracht sein. Die Zahnradsegmente greifen für die Führung ineinander ein, während die Hebel ähnlich geführt sind wie die die Zahnräder, aber seitlich versetzt voneinander. Insbesondere weist der eine Hebel einen Führungssteg auf, der in ein Langloch des anderen Hebels eingreift.

### Kurze Beschreibung der Zeichnungen

Im Folgenden wird die Erfindung mit Bezug auf die Zeichnungen näher erklärt. Es zeigen:
- Fig. 1: a) eine perspektivische Darstellung einer erfindungsgemässen Vorrichtung für einen Nutzer;
b) eine Detailansicht der Vorrichtung aus a), im Bereich eines Gelenks;
c) eine Blende zur Anbringung an ein Traggestell;
- Fig. 2: die Vorrichtung aus Fig. 1 in Seitenansicht
a) bei horizontaler Kopfstellung;
b) bei nach oben geneigter Kopfstellung;
c) bei nach unten geneigter Kopfstellung;
d) bei horizontaler Kopfstellung mit zurückversetzter Lage der Gegengewichte, ein grosses Moment auf das Gelenk erzeugend;
e) bei horizontaler Kopfstellung mit vorversetzter Lage der Gegengewichte, ein kleines Moment auf das Gelenk erzeugend;

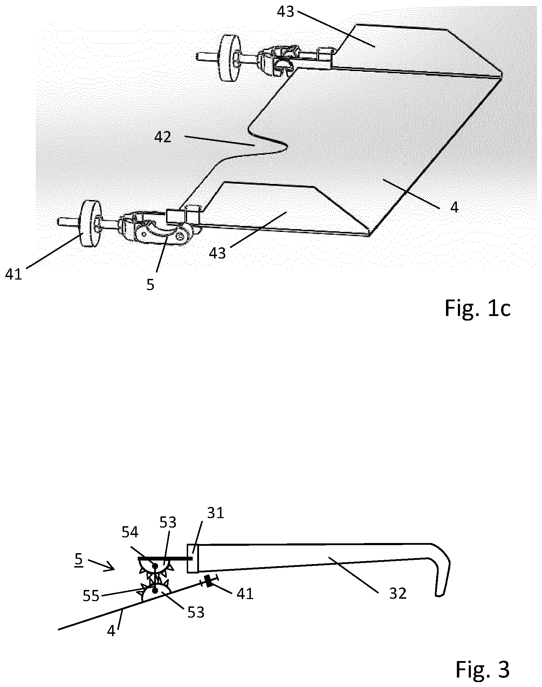

- Fig. 3: Vorrichtung mit einer alternativen Ausführung des Gelenks in einer schematischen Darstellung;

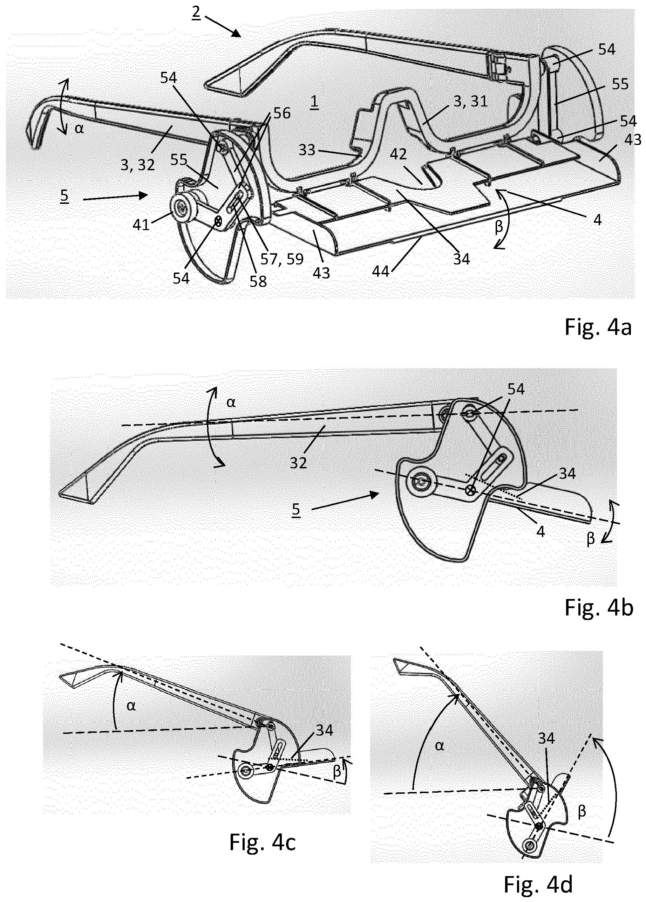

- Fig. 4: Vorrichtung mit einer weiteren alternativen Ausführung des Gelenks
a) in perspektivischer Ansicht;
b) in Seitenansicht in der Grundhaltung;
c) in Seitenansicht mit wenig abgesenktem Kopf;
d) in Seitenansicht mit stark abgesenktem Kopf;

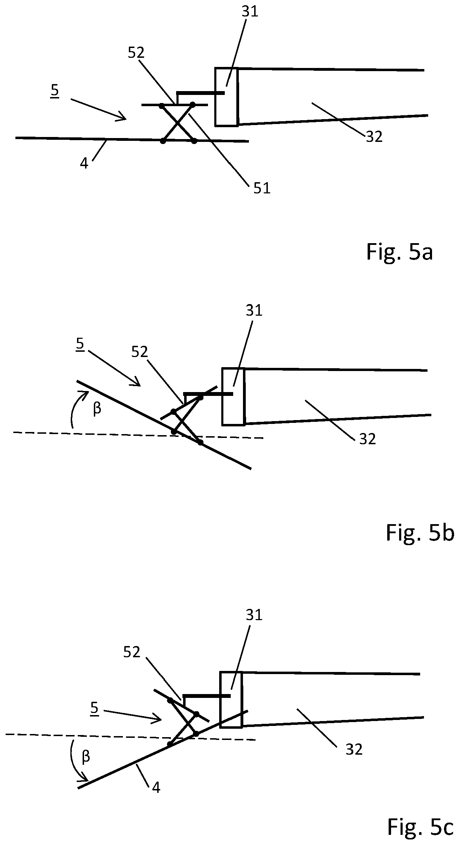

- Fig. 5: eine schematische Darstellung der Vorrichtung in Seitenansicht im Bereich eines neigbaren Gelenks
a) mit dem neigbaren Gelenk in Grundstellung
b) mit nach vorne geneigtem Gelenk;
c) mit nach hinten geneigtem Gelenk.

### Wege zur Ausführung der Erfindung

Die Figuren sind teilweise schematisch angefertigt und lassen keine Schlüsse auf die bevorzugten Winkelstellungen zu.

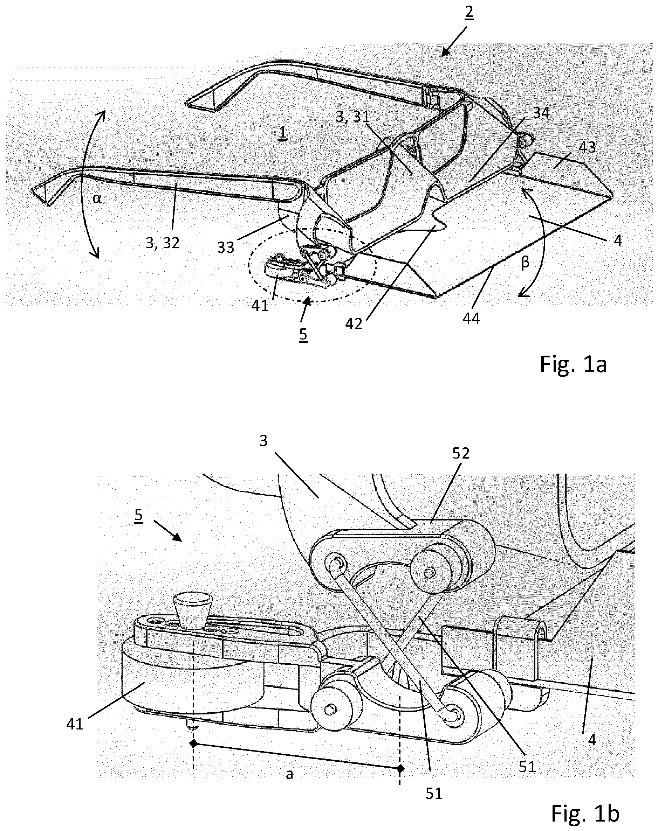

Fig. 1a zeigt eine erfindungsgemässe Vorrichtung 2 für einen Benutzer 1, der selbst nicht dargestellt ist. Diese Vorrichtung 2 wird wie eine Brille am Kopf 1 eines Benutzers getragen und umfasst ein Traggestell 3, das einem Brillengestell ähnelt, sowie beidseitig am vorderen Ende des Traggestells 3 angeordnete Gelenke 5 und eine daran schwenkbar angebrachte Blende 4. In dieser Darstellung sind zudem optionale Brillengläser eingesetzt. Fig. 1c zeigt eine perspektivische Darstellung von der Blende 4, mit dem daran angebrachten unteren Teil des Gelenks 5. Ein Gelenk 5 ist zudem in Fig. 1b vergrössert dargestellt.

Die hier verwendeten Begriffe oben, unten, rechts, links, vorne und hinten beziehen sich auf die Richtungen aus der Sicht des Benutzers 1. «Vorne» ist in Sichtrichtung, «hinten» entsprechend in Richtung hinter den Benutzer.

Das Traggestell 3 umfasst einen Gesichtsbügel 31 sowie zwei Seitenbügel 32 und unterscheidet sich von einer herkömmlichen Brille dadurch, dass am Gesichtsbügel 31 keine Gläser angebracht sein müssen. Das Traggestell 3 stützt sich demnach, wie eine herkömmliche Brille, auf den beiden Ohren und der Nase eines Benutzers 1 ab. Der Gesichtsbügel 31 erstreckt sich über und neben der Nase eines Benutzers 1 entlang der Backenknochen unterhalb der Augenbereiche zu den Seitenbügeln 32, sodass der Benutzer 1 dort, wo bei herkömmlichen Brillen die Gläser eingesetzt wären, freie Sicht hat. Optional können Korrekturgläser eingesetzt werden. Die Seitenbügel 32 sind seitlich am Gesichtsbügel 31 angebracht, beispielsweise klappbar, damit die Vorrichtung platzsparend versorgt werden kann.

Die Blende 4, auch dargestellt in Fig. 1c, steht unterhalb der Augenbereiche nach vorne ab und weist vorzugsweise hinter der Anbringung an den Gelenken 5 Gegengewichte 41 auf. Erfindungsgemäss sind die Gelenke 5 derart ausgestaltet, dass sie bei einer Neigung α des Traggestells 3 eine Neigung β der Blende 4 in Gegenrichtung erzwingen: Verändert sich α in Uhrzeigersinn, muss sich β in Gegenuhrzeigersinn verändern, und umgekehrt. Vorzugsweise weist die Blende 4 im Bereich der Nase eine Ausbuchtung und/oder Aussparung 42 auf, um nicht an der Nase anzustossen.

In einer bevorzugen Ausgestaltung, insbesondere zum Abdecken der an den Rändern gelegenen Tasten eines Klaviers, weist die Blende 3 an den seitlichen Rändern Auskragungen 43 nach oben auf, um das Sichtfeld nach links und rechts aussen entsprechend einzuschränken. Es wird hier darauf hingewiesen, dass alle hier beschriebenen Merkmale miteinander und insbesondere mit verschiedenen Ausführungen kombinierbar sind, auch wenn sie nur in Zusammenhang mit einer der alternativen Ausführungen beschrieben sind.

Sobald ein Benutzer 1 den Kopf nach unten senkt und versucht, dadurch einen Blick auf die Tastatur zu bekommen, schwenkt die Blende 4 nach oben und verdeckt sogleich die erhoffte Sicht nach unten. Somit wirkt die Vorrichtung 2 als Sichtblende, indem das untere Sichtfeld eines Benutzers 1 abgedeckt wird, ungeachtet der aktuellen Neigung des Kopfes des Benutzers 1.

In Fig. 1a sind am Traggestell 3 optionale Gesichtsanpassungen 33 im Bereich der Nase und der Backenknochen sichtbar, die sich zum Gesicht hin erstrecken, um die Sicht unter dem Gesichtsbügel 31 hindurch zu verhindern. Diese sind vorzugsweise weich ausgestaltet, damit sie sich an jede Gesichtsform gut anpassen können.

Die erfindungsgemässe Vorrichtung 2 ist im Gegensatz zu anderen eingangs erwähnten Blenden, die aus belastbarem Papier hergestellt sind, viel handlicher und benutzerfreundlicher zum Tragen. Die Hände und Arme sind durch die Vorrichtung 2 in keiner Weise eingeschränkt.

Zudem wird der Benutzer 1 schnell lernen, dass es hoffnungslos ist, freie Sicht auf die Hände zu bekommen, selbst bei gesenktem Kopf. Der Lernerfolg beim Eintrainieren des Blindschreibens oder Blindspielens wird dadurch erhöht.

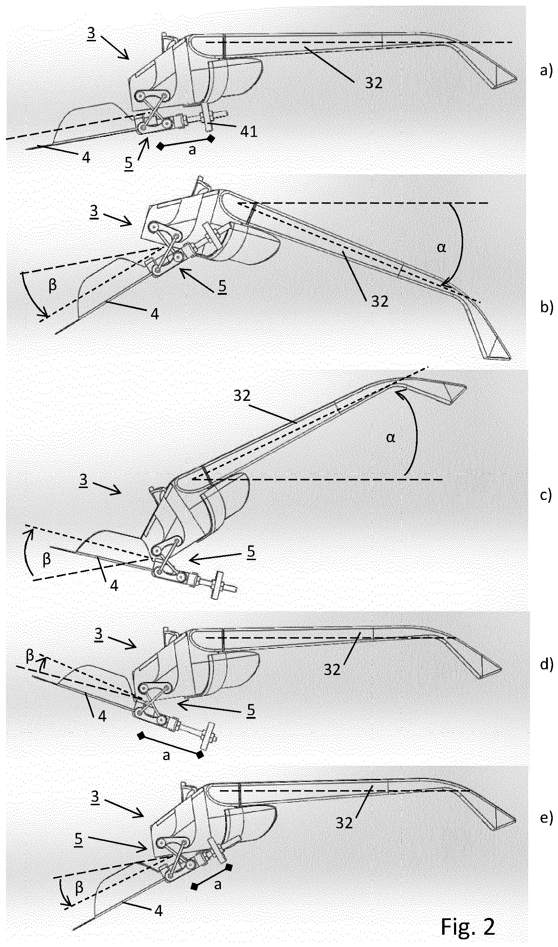

Kernstück der Vorrichtung 2 sind die Gelenke 5. In einer ersten bevorzugten Version, dargestellt in Fig. 1 und 2, umfasst jedes Gelenk 5 zwei sich in einem X kreuzende Stege 51, die an ihren Enden je am Traggestell 3 und an der Blende 4 schwenkbar angebracht sind, sodass die Blende 4 am Traggestell 3 schwenkbar hängt. Die Stege 51 bewegen sich bei einer Kopfneigung in der Neigungsebene.

In Fig. 2a schaut der Benutzer 1 geradeaus, die Seitenbügel 32 sind etwa horizontal ausgerichtet, gekennzeichnet durch eine lang gestrichelte Linie. Die Blende 4 ist in ihrer Grundstellung, welche leicht nach unten geneigt ist. Auch diese Stellung ist durch eine lang gestrichelte Linie gekennzeichnet, ausgehend vom Gelenk 5.

In Fig. 2b schaut der Benutzer 1 mit den Seitenbügeln 32 in einem Winkel α nach oben, ein Neigungswinkel β der Blende 4 bildet sich in Gegenrichtung, die Blende 4 senkt sich vorne ab. Die neuen Neigungsstellungen von Seitenbügel 32 und Blende 4 sind jeweils mit kurz gestrichelten Linien angegeben.

In Fig. 2c ist der Kopf des Benutzers 1 mit den Seitenbügeln 32 im Neigungswinkel α nach unten gesenkt, der Neigungswinkel β der Blende 4 bildet sich in Gegenrichtung, sodass die Blende 4 die Sicht nach unten versperrt.

In Fig. 2 d) und e) wird der Abstand a vom Gelenk 5 zum Gegengewicht 41 versetzt, bei jeweils horizontal gehaltenem Seitenbügel 32. Diese Darstellungen sind mit der Figur 2 a) zu vergleichen. Dort ist das Gegengewicht 41 in einem mittleren Abstand a zum Gelenk 5 angebracht. In Fig. 2 d) wird dieser Abstand a vergrössert, die Blende 4 hebt sich, während bei Fig. 2 e) das Gegenteil der Fall ist.

Die in Fig. 2 dargestellten Winkelverhältnisse α und β dienen als Anschauungsbeispiele.

In Fig. 3 ist das Gelenk 5 in einer alternativen Ausführung schematisch dargestellt. Anstelle der Stege 51 sind hier Zahnradsegmente 53 jeweils drehfest sowohl am Traggestell 3, insbesondere am Gesichtsbügel 31, wie auch an der Blende 4 angeordnet. Sie sind an ihren Achsen 54 mit Distanzhaltern 55 schwenkbar verbunden und greifen ineinander ein. Auch hier ist die Blende 4 somit schwenkbar am Traggestell 3 angebracht und auch hier hebt sich der vordere Bereich der Blende 4, wenn der Kopf mit dem Traggestell 3 nach vorne abgesenkt wird, und umgekehrt. Die Zahnradsegmente 53 erzwingen diese Bewegungen.

In Fig. 4 ist eine Vorrichtung wie in Fig. 1 dargestellt mit einer alternativen Ausgestaltung der Gelenke 5, die den Gelenken in Fig. 3 prinzipiell sehr ähnlich sind. Auch in dieser Ausführung umfasst jedes Gelenk 5 einerseits einen Distanzhalter 55, welcher an je einer Achse 54 am Traggestell 3 und an der Blende 4 schwenkbar angebracht ist, sodass die Blende 4 am Traggestell 3 schwenkbar hängt. In dieser Ausführung ist der Distanzhalter 55 als Platte ausgestaltet, welche zwei Durchgänge für die Achsen 54 aufweist. Anstelle der Zahnradsegmente 53 sind hier aber zwei Hebel 56 angebracht. Jeder dieser Hebel 56 ist im Bereich der Achsen 54 mit dem Traggestell 3 resp. mit der Blende 4 drehfest verbunden. Sie greifen durch eine Führung 57 beweglich derart ineinander ein, dass auf Grund der Neigung α des Traggestells 3 die Neigung β der Blende 4 bestimmt wird, wie in Fig. 2 erläutert.

Dieses System kann als Spezialausführung der Ausführung mit den Zahnradsegmenten 53 angesehen werden. Der obere Hebel 56 repräsentiert einen Zahn eines oberen Zahnradsegmentes 53, während die Aussparung zwischen zwei Zähnen des unteren Zahnradsegmentes 53 durch eine Aussparung im unteren Hebel 56 ausgestaltet ist. Dieser kann dazu wie eine Stimmgabel ausgestaltet sein, also mit einem endseitig offen Langloch 58, oder ein geschlossenes Langloch 58 aufweisen, wie in Fig. 3a dargestellt. Der obere Hebel 56 ist aber, im Gegensatz zur Ausführung mit den Zahnradsegmenten 53, seitlich versetzt zum unteren Hebel 56 angeordnet und greift mit einem Führungssteg 59 in das offene oder geschlossene Langloch 58 ein. Alternativ zur dargestellten Vorrichtung 2 kann der Führungssteg 59 auch beidseitig des Langlochs 58 am Hebel 56 befestigt sein. Kräfte zwischen den Hebeln 56 werden nur in der Ebene der Neigungswinkel α, β übertragen, nicht quer dazu.

Wichtig ist, dass die verwendeten Gelenke 5 reibungsarm sind, damit die Reaktion der Blende 4 auf eine veränderte Kopfstellung unmittelbar erfolgt. Bei den hier beschriebenen Gelenken 5 ändert die Neigung β der Blende 4 auf Grund einer Änderung des Neigungswinkels α der Kopfstellung, da sich die Blende 4 entsprechend der Anbringung am Gelenk 5 neu ausrichtet. Die in Fig. 4 dargestellten plattenförmigen Distanzhalter 55 wie auch die stabförmigen Distanzhalter 55 in Fig. 3 sind daher locker an den Achsen 54 der Zahnradsegmente 53 resp. der Hebel 56 angebracht. Da die Distanzhalter 55 aus energetischen Gründen die senkrechte Stellung bevorzugen, damit der Schwerpunkt der Blende 4 seine tiefst mögliche Lage einnehmen kann, erzwingen sie bei einer Neigung des Kopfes eines Benutzers 1 eine Neigung der Blende 4 in Gegenrichtung.

Auch in der Fig. 4a sind am Traggestell 3 optionale, vorzugsweise weiche Gesichtsanpassungen 33 im Bereich der Nase und der Backenknochen sichtbar, die sich zum Gesicht hin erstrecken, um die Sicht unter dem Gesichtsbügel 31 hindurch zu verhindern.

Zudem ist in dieser Ausführung am Traggestell 3 unter den Augen und hier auch im Bereich der Nase ein Schild 34 angebracht, welches die Sicht zwischen der Blende 4 und dem Traggestell 3 hindurch verhindert. Diese kann schwenkbar am Gesichtsbügel 31 angebracht sein und sich an seinem vorderen Ende auf der Blende 4 abstützten. In den Figuren 4b bis 4d ist das nicht sichtbare Schild 34 gepunktet dargestellt.

Vorzugsweise ist, unabhängig von der Ausgestaltung des Gelenks 5, eine vordere Kante 44 der Blende 4 in einem variablen Abstand zum Traggestell 3 positionierbar. Sie kann insbesondere ausklappbar, ausziehbar oder in Faltungen verlängerbar sein. Diese Kante 44 definiert den Sichtwinkel nach unten. Je weiter sie vorgerückt ist, desto mehr verdeckt sie die Sicht nach unten. Eine variable vordere Kante 44 ist daher auch hilfreich zum Einstellen der Grundstellung der Brille. Da sich der Schwerpunkt der Blende 4 durch eine Verschiebung der Kante 44 verändern kann, sollten die Abstände a der Gegengewichte 41 zu den Gelenken 5 angepasst werden.

Es ist zu beachten, dass Benutzer, insbesondere Klavierschüler sehr unterschiedliche Grössen aufweisen können, die entsprechend auch unterschiedlich eingestellte Traggestelle benutzen müssen, um den erwünschten Erfolg zu erzielen.

Die Blende 4 wird möglichst in ihrem Schwerpunkt aufgehängt, damit sie kein Moment auf das Gelenk 5 ausübt. Dazu können die Gegengewichte 41 in einem verschiebbaren Abstand a zu den Gelenken 5 angeordnet sein. Je nach Grösse und Sitzhöhe des Benutzers 1 und je nach Position der Tastatur muss die Grundeinstellung des Neigungswinkels β der Blende 4 angepasst werden. Durch die Verschiebung dieser Gegengewichte 41 und somit durch die Veränderung des Abstandes a zwischen den Gegengewichten 41 und den Gelenken 5 wird diese Grundeinstellung verändert und individuell eingestellt. Natürlich ist der physikalische Effekt identisch, wie wenn die Gegengewichte 41 schwerer oder leichter werden, aber an Ort bleiben. Es ist aber einfacher, Gegengewichte 41 zu verschieben, als Zusatz- oder Austauschgewichte bereitzustellen.

In der bevorzugten Ausführung nach Fig. 3 können die Gelenke 5 in ihrer Grundposition auch durch Änderung ihrer Zahnradstellung der beiden Zahnradsegmente 53 zueinander verändert werden. Dazu könnten die Distanzhalter 55 nach Überwindung einer Federkraft teleskopartig auseinandergezogen werden, oder ein seitlicher Versatz ist denkbar, um nur zwei Beispiele zu nennen.

In der bevorzugten Ausführung nach Fig. 4 können zudem die Winkel, welche die Hebel 56 zum Tragbügel 3 resp. zur Blende 4 einnehmen verstellbar sein, um die Grundstellung der Blende 4 zu verändern.

In einer weiteren alternativen Ausführung, systematisch dargestellt in Fig. 5, sind entweder die oberen oder die unteren der jeweils drehfest angebrachten Teile der Gelenke 5 an schwenkbaren Haltevorrichtungen 52 am Traggestell 3, insbesondere an den Enden des Gesichtsbügels 31, resp. an der Blende 4 angebracht. Hier ist das Gelenk 5 nach Fig. 1 und 2 dargestellt, analog können auch die Ausführungen nach Fig. 3 oder 4 so ausgestaltet sein.

In allen Figuren 5a bis 5c ist der Neigungswinkel α des Seitenbügels 32 jeweils 0°, der Benutzer 1 schaut horizontal. In Fig. 5a) ist die Haltevorrichtung 52 des oberen Gelenks 5 derart eingestellt, dass die Blende 4 etwa horizontal steht, während die Haltevorrichtungen 52 in den Figuren 5b und 5c die oberen Teile der Gelenke 5 in geneigter Stellung fixieren. Die Blende 4 wird dadurch jeweils in einem Winkel β zur Horizontalen in Fig. 5b nach oben, resp. in Fig. 5c nach unten geneigt, ohne dass sich dafür der Neigungswinkel α der Seitenbügel 32 veränderte. So lässt sich die Grundeinstellung der Neigung der Blende 4 ohne Verstellung oder Veränderung von Gegengewichten 41 nach Bedarf justieren. Gegengewichte 41 sollten dennoch vorhanden sein, sie sorgen stets dafür, dass die Blende im Gleichgewicht ist, indem das Gewicht der Blende 4 kein Moment auf das Gelenk 5 erzeugt. Die Gelenke 5 an den schwenkbaren Haltevorrichtungen 52 gemäss Fig. 5 können auch als Zahnradsegmente 53 wie in Fig. 3 oder als Hebel 56 wie in Fig. 4 ausgestaltet sein. Auch diese Gelenke 5 lassen sich justieren, indem eine ihrer Haltevorrichtungen 52 geneigt wird. Während dem Gebrach sind die Zahnradsegmente 53 resp. die Hebel 56 aber stets drehfest mit dem Traggestell 3 resp. mit der Blende 4 verbunden.

Die Anbringung der Gelenke 5 seitlich am Gesichtsbügel 31, der stets bis zu den Seitenbügeln 32 reicht, hat den Vorteil, dass sich die Seitenbügel 32 wie bei einer normalen Brille einklappen lassen. Eine Anbringung der Gelenke 5 an den Seitenbügeln 32 lässt dies nur mit zusätzlichem Aufwand zu.

Es hat sich als zweckmässig erwiesen, dass die Grundeinstellung der Blende 4 derart justiert wird, dass die Sicht nach unten nur bis zu einem Winkel zwischen 30 und 60° gegenüber der Horizontalen, insbesondere etwa um die 45° ± 5° gewährleistet ist, unabhängig von der Kopfneigung. Darunter sollte die Sicht durch die Blende abgedeckt sein.

### Bezugszeichenliste

- 1: Ort eines Benutzers, Ort des Kopfs eines Benutzers
- 2: Vorrichtung
- 3: Traggestell
- 31: Gesichtsbügel
- 32: Seitenbügel
- 33: Gesichtsanpassung
- 34: Schild
- 4: Blende
- 41: Gegengewicht
- 42: Ausbuchtung und/oder Aussparung
- 43: Auskragung
- 44: vordere Kante der Blende
- 5: Gelenk
- 51: Steg
- 52: Haltevorrichtung, neigbar
- 53: Zahnradsegment
- 54: Achse, Achse des Zahnrades
- 55: Distanzhalter, Platte
- 56: Hebel
- 57: Führung
- 58: Langloch
- 59: Führungssteg
- α: Neigungswinkel des Traggestells resp. der Kopfstellung des Benutzers
- β: Neigungswinkel der Blende
- a: Abstand

## Patentansprüche

1. Am Kopf eines Benutzers (1) tragbare Vorrichtung (2) als Sichtblende zum Abdecken des unteren Sichtfeldes, ungeachtet der aktuellen Kopfneigung, welche einem Benutzer (1) beim Tragen beispielsweise die Sicht auf eine Schreibtastatur oder zu den Tasten eines Klaviers verdeckt, umfassend ein wie ein Brillengestell tragbares Traggestell (3) sowie eine flächig ausgestaltete Blende (4), welche unterhalb der Augenbereiche nach vorne in Sehrichtung absteht, **dadurch gekennzeichnet, dass** die Blende (4) seitlich am Traggestell (3) schwenkbar an Gelenken (5) angebracht ist, wobei die Gelenke (5) derart ausgestaltet sind, dass sie bei einer Neigung des Traggestells (3) eine Neigung der Blende (4) in Gegenrichtung erzwingen.

2. Vorrichtung (2) nach Anspruch 1 **dadurch gekennzeichnet, dass** an der Blende (4) hinter den Gelenken (5), entgegen der Sehrichtung, Gegengewichte (41) angebracht sind.

3. Vorrichtung (2) nach Anspruch 2, **dadurch gekennzeichnet, dass** die Gegengewichte (41) in einem beweglichen Abstand (a) zu den Gelenken (5) angeordnet sind und dass sich eine gewünschte Grundeinstellung des Neigungswinkels (β) der Blende (4) durch Verschieben der Gegengewichte einstellen lässt.

4. Vorrichtung nach einem der vorhergehenden Ansprüche, **dadurch gekennzeichnet, dass** die Gelenke (5) am Traggestell (3) oder an der Blende (4) an neigbaren Haltevorrichtungen (52) angebracht sind und dass sich eine gewünschte Grundeinstellung des Neigungswinkels (β) der Blende (4) durch Verstellen der Neigung der Haltevorrichtungen (52) einstellen lässt.

5. Vorrichtung (2) nach einem der Ansprüche 1 bis 4, **dadurch gekennzeichnet, dass** jedes Gelenk (5) zwei sich in einem X kreuzende Stege (51) umfasst, die an ihren Enden je am Traggestell (3) und an der Blende (4) schwenkbar angebracht sind, sodass die Blende (4) am Traggestell (3) schwenkbar hängt.

6. Vorrichtung (2) nach einem der Ansprüche 1 bis 4, **dadurch gekennzeichnet, dass** jedes Gelenk (5) ineinander eingreifende Zahnradsegmente (53) umfasst, von denen je eines drehfest am Traggestell (3) resp. an der Blende (4) angebracht ist, wobei die Zahnradsegmente (53) an ihren Achsen (54) mit Distanzhaltern (55) schwenkbar miteinander verbunden sind, sodass die Blende (4) am Traggestell (3) schwenkbar hängt.

7. Vorrichtung (2) nach einem der Ansprüche 1 bis 4, **dadurch gekennzeichnet, dass** jedes Gelenk (5) einerseits einen Distanzhalter (55) umfasst, welcher an je einer Achse (54) am Traggestell (3) und an der Blende (4) schwenkbar angebracht ist, sodass die Blende (4) am Traggestell (3) schwenkbar hängt, sowie andererseits zwei Hebel (56) umfasst, die jeweils im Bereich der Achsen (54) mit dem Traggestell (3) resp. mit der Blende (4) drehfest verbunden sind und durch eine Führung (57) beweglich derart ineinander eingreifen, dass dadurch die Neigung der Blende (4) auf Grund der Neigung des Traggestells (3) bestimmt wird.

8. Vorrichtung nach Anspruch 7, **dadurch gekennzeichnet, dass** die Führung (57) dadurch erreicht wird, dass einer der Hebel (56) mit einem Langloch (58) und der andere mit einem darin geführten Führungssteg (60) versehen ist.

9. Vorrichtung (2) nach einem der vorhergehenden Ansprüche, **dadurch gekennzeichnet, dass** am Traggestell (3) Gesichtsanpassungen (33) im Bereich der Nase und/oder der Backenknochen angebracht sind, die sich zum Gesicht hin erstrecken, um die Sicht unter dem Traggestell (3) hindurch zu verhindern.

10. Vorrichtung (2) nach einem der vorhergehenden Ansprüche, **dadurch gekennzeichnet, dass** am Traggestell (3) im Bereich unter den Augen ein Schild (34) angebracht ist, welches die Sicht zwischen der Blende (4) und dem Traggestell (3) hindurch verhindert.

11. Vorrichtung (2) nach Anspruch 10, **dadurch gekennzeichnet, dass** das Schild (34) am Traggestell (3) schwenkbar angebracht ist und sich an seinem vorderen Ende auf der Blende (4) abstützt.

12. Vorrichtung (2) nach einem der vorhergehenden Ansprüche, **dadurch gekennzeichnet, dass** die Blende (4) eine vordere Kante (44) aufweist, deren Abstand zum Traggestell (3) variabel einstellbar ist.

13. Vorrichtung (2) nach einem der vorhergehenden Ansprüche, **dadurch gekennzeichnet, dass** die Blende (4) und/oder ggf. das Schild (34) im hinteren, mittigen Bereich eine Ausbuchtung (42) nach oben und/oder eine Aussparung aufweist, um beim Tragen nicht an der Nase anzustehen.

14. Vorrichtung (2) nach einem der vorhergehenden Ansprüche, **dadurch gekennzeichnet, dass** die Blende (4) an den seitlichen Rändern Auskragungen (43) nach oben aufweist, um beim Tragen das Sichtfeld nach links und rechts aussen weiter einzuschränken.

15. Vorrichtung (2) nach einem der vorhergehenden Ansprüche, **dadurch gekennzeichnet, dass** die Grundeinstellung der Blende 4 derart justierbar ist, dass die Sicht nach unten nur bis zu einem Winkel zwischen 30 und 60° gegenüber der Horizontalen, insbesondere nur bis 45° ± 5° gewährleistet ist.

## Claims

1. A device (2) in the form of a screen that can be worn on the head of a user (1) and serves for covering the lower field of vision regardless of the current head inclination, wherein said device obscures the view, for example, of a keyboard or the keys of the piano for a user (1) when worn and comprises a supporting frame (3) that can be worn like a spectacle frame and a flat blind (4) that protrudes forward in the viewing direction underneath the eye regions, **characterized in that** the blind (4) is pivotably mounted laterally on the supporting frame (3) at joints (5), wherein the joints (5) are designed in such a way that they force the blind (4) to tilt in the opposite direction when the supporting frame (3) is tilted.

2. The device (2) according to claim 1, **characterized in that** counterweights (41) are mounted on the blind (4) behind the joints (5) opposite to the viewing direction.

3. The device (2) according to claim 2, **characterized in that** the counterweights (41) are arranged at a movable distance (a) from the joints (5), and **in that** a desired basic adjustment of the angle of inclination (β) of the blind (4) can be adjusted by displacing the counterweights.

4. The device according to one of the preceding claims, **characterized in that** the joints (5) are mounted on the supporting frame (3) or on the blind (4) on inclinable holding devices (52), and **in that** a desired basic adjustment of the angle of inclination (β) of the blind (4) can be adjusted by altering the inclination of the holding devices (52).

5. The device (2) according to one of claims 1 to 4, **characterized in that** each joint (5) comprises two bars (51) that intersect one another in the form of an X, wherein the respective ends of said bars are pivotably mounted on the supporting frame (3) and on the blind (4) such that the blind (4) is pivotably suspended on the supporting frame (3).

6. The device (2) according to one of claims 1 to 4, **characterized in that** each joint (5) comprises gearwheel segments (53) that engage into one another, wherein one of said gearwheel segments is respectively mounted on the supporting frame (3) and on the blind (4) in a rotationally fixed manner, and wherein the gearwheel segments (53) are on their axes (54) pivotably connected to one another with spacers (55) such that the blind (4) is pivotably suspended on the supporting frame (3).

7. The device (2) according to one of claims 1 to 4, **characterized in that** each joint (5) on the one hand comprises a spacer (55), which is pivotably mounted on the supporting frame (3) and on the blind (4) at a respective axis (54) such that the blind (4) is pivotably suspended on the supporting frame (3), and **in that** each joint on the other hand comprises two levers (56), which are respectively connected to the supporting frame (3) and to the blind (4) in a rotationally fixed manner in the region of the axes (54) and movably engage into one another by means of a guide (57) in such a way that the inclination of the blind (4) is thereby defined based on the inclination of the supporting frame (3).

8. The device according to claim 7, **characterized in that** the guide (57) is realized **in that** one of the levers (56) is provided with an oblong hole (58) and the other lever is provided with a guide bar (60) that is guided in the said oblong hole.

9. The device (2) according to one of the preceding claims, **characterized in that** face adaptations (33) are mounted on the supporting frame (3) in the region of the nose and/or the cheekbones and extend toward the face in order to obscure the view underneath the supporting frame (3).

10. The device (2) according to one of the preceding claims, **characterized in that** a shield (34) is mounted on the supporting frame (3) in the region underneath the eyes and obscures the view between the blind (4) and the supporting frame (3).

11. The device (2) according to claim 10, **characterized in that** the shield (34) is pivotably mounted on the supporting frame (3) and supported on the blind (4) at its front end.

12. The device (2) according to one of the preceding claims, **characterized in that** the blind (4) has a front edge (44), the distance of which from the supporting frame (3) is variably adjustable.

13. The device (2) according to one of the preceding claims, **characterized in that** the blind (4) and/or optionally the shield (34) has an upward bulge (42) and/or a recess in the rear central region so as not to rest against the nose when the device is worn.

14. The device (2) according to one of the preceding claims, **characterized in that** the blind (4) has upwardly extending projections (43) on the lateral edges in order to further restrict the field of vision to the left and the right when the device is worn.

15. The device (2) according to one of the preceding claims, **characterized in that** the basic adjustment of the blind 4 can be adjusted in such a way that the downward view is only ensured up to an angle between 30 and 60°, particularly only up to 45° ± 5°, in relation to the horizontal.

## Revendications

1. Dispositif (2), susceptible d'être porté sur la tête d'un utilisateur (1), pour servir de visière, destinée à recouvrir le champ de vision inférieur, quelle que soit l'inclinaison actuelle de la tête, lequel occulte pour un utilisateur (1) qui le porte, par exemple la vision sur clavier ou sur les touches d'un clavier, comprenant une monture porteuse (3) susceptible d'être portée comme une monture de lunettes, ainsi qu'un écran (4) conçu de forme plane, lequel déborde vers l'avant dans la direction de vision, en-dessous de la zone oculaire, **caractérisé en ce que** l'écran (4) est monté latéralement sur la monture porteuse (3) de manière pivotante sur des articulations (5), les articulations (5) étant conçues de telle sorte, que lors d'une inclinaison de la monture porteuse (3), elles forcent une inclinaison de l'écran (4) dans la direction opposée.

2. Dispositif (2) selon la revendication 1, **caractérisé en ce que** sur l'écran (4), des contrepoids (41) sont montés derrière les articulations (5), à l'encontre de la direction de vision.

3. Dispositif (2) selon la revendication 2, **caractérisé en ce que** les contrepoids (41) sont placés à un écart (a) mobile par rapport aux articulations (5) et **en ce qu'**un réglage de base souhaité de l'angle d'inclinaison (β) de l'écran (4) peut se régler par un coulissement des contrepoids.

4. Dispositif selon l'une quelconque des revendications précédentes, **caractérisé en ce que** les articulations (5) sont montées sur la monture porteuse (3) ou sur l'écran (4) sur des dispositifs de support (52) inclinables et **en ce qu'**un réglage de base souhaité de l'angle d'inclinaison (β) de l'écran (4) peut être réglé par l'ajustement de l'inclinaison des dispositifs de support (52).

5. Dispositif (2) selon l'une quelconque des revendications 1 à 4, **caractérisé en ce que** chaque articulation (5) comporte deux barrettes (51) se croisant en X, qui par leurs extrémités sont montées de manière pivotante chacune sur la monture porteuse (3) et sur l'écran (4), de sorte que l'écran (4) soit accroché de manière pivotante sur la monture porteuse (3) .

6. Dispositif (2) selon l'une quelconque des revendications 1 à 4, **caractérisé en ce que** chaque articulation (5) comprend des segments dentés (53) s'engrenant les uns dans les autres, dont chaque fois l'un est monté de manière solidaire en rotation sur la monture porteuse (3), respectivement sur l'écran (4), les segments dentés (53) étant reliés les uns avec les autres sur leurs axes (54) avec des espaceurs (55), de sorte que l'écran (4) soit accroché de manière pivotante sur la monture porteuse (3).

7. Dispositif (2) selon l'une quelconque des revendications 1 à 4, **caractérisé en ce que** chaque articulation (5) comprend d'une part un espaceur (55), lequel est monté de manière pivotante sur chaque fois un axe (54) sur la monture porteuse (3) et sur l'écran (4), de sorte que l'écran (4) soit accroché de manière pivotante sur la monture porteuse (3), ainsi que d'autre part, deux leviers (56), dont chacun est assemblé dans la zone des axes (54) avec la monture porteuse (3), respectivement avec l'écran (4) et qui s'engrènent l'un dans l'autre de manière mobile par un guidage (57), de sorte à déterminer de ce fait l'inclinaison de l'écran (4) sur la base de l'inclinaison de la monture porteuse (3).

8. Dispositif selon la revendication 7, **caractérisé en ce que** le guidage (57) est obtenu **en ce que** l'un des leviers (56) est muni d'un trou oblong (58) et **en ce que** l'autre est muni d'une barrette de guidage (60) guidée dans celui-ci.

9. Dispositif (2) selon l'une quelconque des revendications précédentes, **caractérisé en ce que** sur la monture porteuse (3) sont montés dans la zone du nez et / ou des pommettes des adaptateurs faciaux (33) qui s'étendent en direction du visage, pour empêcher la vision à travers le bas de la monture porteuse (3).

10. Dispositif (2) selon l'une quelconque des revendications précédentes, **caractérisé en ce que** sur la monture porteuse (3), dans la zone sous les yeux est monté un obturateur (34), lequel empêche la vision entre l'écran (4) et la monture porteuse (3).

11. Dispositif (2) selon la revendication 10, **caractérisé en ce que** l'obturateur (34) est monté de manière pivotante sur la monture porteuse (3) et s'appuie par son extrémité antérieure sur l'écran (4).

12. Dispositif (2) selon l'une quelconque des revendications précédentes, **caractérisé en ce que** l'écran (4) comporte une arête (44) antérieure, dont l'écart par rapport à la monture porteuse (3) est réglable de manière variable.

13. Dispositif (2) selon l'une quelconque des revendications précédentes, **caractérisé en ce que** l'écran (4) et / ou le cas échéant l'obturateur (34) comporte dans la zone postérieure centrale une protubérance (42) vers le haut et / ou une encoche, pour ne pas reposer sur le nez, lors du port.

14. Dispositif (2) selon l'une quelconque des revendications précédentes, **caractérisé en ce que** l'écran (4) comporte sur les bords latéraux des saillies (43) vers le haut, pour restreindre encore à l'extérieur le champ de vision vers la gauche et vers la droite, lors du port.

15. Dispositif (2) selon l'une quelconque des revendications précédentes, **caractérisé en ce que** le réglage de base de l'écran (4) est ajustable de telle sorte que la vision vers le bas ne soit assurée que jusqu'à un angle compris entre 30 et 60 ° par rapport à l'horizontale, notamment que jusqu'à 4° ± °.
